# EUROPEAN PATENT SPECIFICATION

(11) **EP 2 700 602 B1**
(45) Date of publication and mention of the grant of the patent: **30.11.2016**
(21) Application number: 12773550.4
(22) Date of filing: 23.02.2012
(51) Int. Cl.: B65H 63/00, G01N 3/08, G01N 33/36, B65H 69/00, D01H 13/32

(54) **YARN WINDING DEVICE, AUTOMATIC WINDER, AND TEXTILE MACHINE**
GARNAUFWICKELVORRICHTUNG, AUTOMATISCHE SPULMASCHINE UND TEXTILMASCHINE
DISPOSITIF DE BOBINAGE DE FIL, BOBINOIR DE FIL AUTOMATIQUE, ET MACHINE TEXTILE

(30) Priority: 19.04.2011 JP 2011093044
(43) Date of publication of application: 26.02.2014
(73) Proprietor: Murata Machinery, Ltd., Kyoto-shi, Kyoto 601-8326 (JP)
(72) Inventor: TANIGAWA Yasunobu, Kyoto-shi Kyoto 612-8686 (JP)
(74) Representative: Zimmermann, Tankred Klaus
(86) International application number: PCT/JP2012/054463
(87) International publication number: WO 2012/144267

(56) References cited:
- EP-A1- 0 405 188
- EP-A1- 1 235 071
- DE-A1- 3 936 517
- DE-A1- 4 004 755
- DE-A1- 4 335 089
- JP-A- 2 243 475
- JP-A- S63 315 625
- JP-A- 2002 173 269
- JP-A- 2010 047 391
- JP-U- H0 376 867
- US-A- 4 733 829
- US-A- 5 235 852

## Description

### Technical Field

The present invention relates to a yarn winding device winding a yarn from a yarn supplying bobbin to form a package, an automatic winder including a plurality of yarn winding devices, and a textile machine including the automatic winder, spinning machine, and other units.

### Background Art

Yarn winding devices producing packages with given specifications by winding yarns from yarn supplying bobbins and forming the packages are publicly known. In such yarn winding devices, splicing is performed to splice a lower yarn from a yarn supplying bobbin and an upper yarn to a package after removing a yarn defect or replacing a yarn supplying bobbin. Note that there is possibility of yarn breaking due to insufficient tensile properties on a spliced part that can occur in downstream processing if the spliced part spliced by splicing has not enough tensile properties.

In a traditional practice, in order to confirm whether tensile properties on a spliced part is enough and stable, sampling a yarn having a spliced part is performed two or more times for each of the yarn winding devices, and measurement to determine whether the tensile properties on the spliced part is equal to or higher than a reference value is performed with a measuring instrument that is separately provided (see Patent Literatures 1 and 2, for example).

EP 1 235 071 A1 describes a process to test the break and stretch characteristics of a sample of yarn. During the manufacturing process, during which a measurement is made, the yarn is spooled from a cops to a cross-wound bobbin. In the process, the thread is laced under tension and the force is measured that is required to break the yarn. The test is repeated at intervals. A measuring assembly is positioned between the cop and the cross-wound bobbin. Following the tear test, the two ends are spliced and spooling continues. The test is undertaken either at the beginning or end of a spool.

### Citation List

### Patent Literature

Patent Literature 1: Japanese Patent Application Laid-Open Publication No. 55-101561
Patent Literature 2: Japanese Examined Patent Application Publication No. 1-24706

### Summary of Invention

### Technical Problem

However, it is typical that one set of the measurement instrument mentioned above is prepared to be shared by the plurality of yarn winding devices. Thus, the measurement of the tensile properties of a yarn sampled is time-consuming task because strict attention is required to associate a yarn winding device with a yarn sampled, for example.

The present invention aims to provide a yarn winding device, automatic winder, and textile machine capable of measuring tensile properties of a yarn readily and accurately.

### Solution to Problem

A yarn winding device of the present invention, winding a yarn from a yarn supplying bobbin to form a package, the yarn winding device comprises a bobbin supporting section supporting the yarn supplying bobbin, a winding section winding the yarn from the yarn supplying bobbin supported with the bobbin supporting section and forming the package, and a tensile properties measuring section measuring tensile properties of the yarn drawn from the yarn supplying bobbin, between the bobbin supporting section and the winding section.

The tensile properties measuring section
measures a breaking intensity of the yarn.

In this yarn winding device, the tensile properties of the yarn drawn from the yarn supplying bobbin is measured with the tensile properties measuring section between the bobbin supporting section and the winding section. As described above, the yarn winding device can measure the tensile properties of the yarn, and thus the tensile properties of the yarn can be measured readily and accurately with the yarn winding device.

The yarn winding device of the present invention further comprises a tension sensor measuring tension of the running yarn being wound
with the winding section, the tensile properties measuring section includes a holding section holding the yarn drawn from the yarn supplying bobbin, a stretching section stretching the yarn held with the holding section, and a tensile properties detecting section detecting the tensile properties of the yarn between the holding section and the stretching section, and the tension sensor functions as the tensile
properties detecting section. With this configuration, the tensile properties of the yarn are detected with the tension sensor. Thus, the number of components can be reduced, and the structure can be simplified. Furthermore, when detecting the tensile properties of the yarn, the yarn drawn from the yarn supplying bobbin is held with the holding section. Thus, the tensile properties measured part can be prevented from being wound into the package (in other words, it is possible to prevent the formation of a package containing the tensile properties measured part).

In the yarn winding device of the present invention, the winding section may function as the stretching section. With this configuration, the yarn is stretched with the winding section. Thus, the number of components can be reduced, and the structure can be simplified.

The yarn winding device of the present invention may further comprise a tension applying section applying tension to the yarn running, between the bobbin supporting section and the winding section, and the tension applying section may function as the holding section. With this configuration, the yarn is held with the tension applying section. Thus, the number of components can be reduced, and the structure can be simplified.

The yarn winding device of the present invention may further comprise a yarn defect passing regulator regulating passage of a yarn defect that is larger than a reference value, with a pair of regulating members arranged in both sides of the yarn running, with a predetermined gap, between the bobbin supporting section and the winding section, and the yarn defect passing regulator may function as the holding section by using the regulating members to hold the yarn. With this configuration, the yarn is held with the yarn defect passing regulator. Thus, the number of components can be reduced, and the structure can be simplified.

An automatic winder of the present invention comprises a plurality of yarn winding devices each winding a yarn from a yarn supplying bobbin to form a package, and each of the yarn winding devices includes a bobbin supporting section supporting the yarn supplying bobbin, a winding section winding the yarn from the yarn supplying bobbin supported with the bobbin supporting section and forming the package, and a tensile properties measuring section measuring tensile properties of the yarn drawn from the yarn supplying bobbin, between the bobbin supporting section and the winding section.

In this automatic winder, the tensile properties of the yarn drawn from the yarn supplying bobbin is measured by the tensile properties measuring section between the bobbin supporting section and the winding section in each of the yarn winding devices. As described above, each yarn winding device can measure the tensile properties of the yarn, and thus the tensile properties of the yarn can be measured readily and accurately with the automatic winder.

In the automatic winder of the present invention, the tensile properties measuring section measures breaking intensity of the
yarn. With this configuration, the tensile properties measurement breaks the yarn. Thus, the tensile properties measured part can be prevented from being wound into the package (in other words, it is possible to prevent the formation of a package containing the tensile properties measured part).

In the automatic winder of the present invention, each of the yarn winding devices may further include a splicer splicing the yarn that has been cut, between the bobbin supporting section and the winding section, and tensile properties measuring section may measure the tensile properties of the yarn in the state with a spliced part spliced with the splicer. With this configuration, the tensile properties of the yarn with a spliced part can be managed numerically and thus, the quality control of the spliced part can be simplified. Furthermore, the splicer may be adjusted on the basis of the tensile properties of the yarn with a spliced part so as to splice yarns with proper tensile properties.

In the automatic winder of the present invention, each of the yarn winding devices may further include a splicer splicing the yarn that has been cut, between the bobbin supporting section and the winding section, and the tensile properties measuring section may measure the tensile properties of the yarn in the state with a spliced part spliced with the splicer and measures the tensile properties of the yarn in the state without the spliced part. With this configuration, the tensile properties of the yarn with a spliced part can be managed numerically and thus, the quality control of the spliced part can be simplified. Furthermore, the splicer may be adjusted on the basis of the tensile properties of the yarn with a spliced part so as to splice yarns with proper tensile properties. In addition to the above, measurement of the tensile properties of the yarn without the spliced part makes it possible to guess which, the tensile properties of the spliced part or the tensile properties of the yarn itself, causes the tensile properties of the yarn with the spliced part.

The automatic winder of the present invention may further comprise a display displaying the tensile properties measured with the tensile properties measuring section. With this configuration, an operator can quickly confirm the tensile properties of the yarn. Furthermore, after the operator adjusts the yarn winding devices, the operator can understand whether the setting-up through the adjustment is proper, for example.

The automatic winder of the present invention may further comprise a control device controlling the yarn winding devices, and the control device may include a managing unit storing and managing the tensile properties measured with the tensile properties measuring section. With this configuration, quality control on packages and the like formed with the yarn winding devices can be practiced advantageously.

A textile machine of the present invention comprises a spinning frame including a plurality of spinning devices each forming a yarn supplying bobbin, an automatic winder including a plurality of yarn winding devices each winding a yarn from the yarn supplying bobbin to form a package, a bobbin feeder feeding the yarn supplying bobbin from the spinning frame to the automatic winder, and a control device controlling at least the automatic winder, each of the yarn winding devices includes a bobbin supporting section supporting the yarn supplying bobbin, a winding section winding the yarn from the yarn supplying bobbin supported with the bobbin supporting section and forming the package, and a tensile properties measuring section measuring tensile properties of the yarn drawn from the yarn supplying bobbin, between the bobbin supporting section and the winding section, and the control device identifies spinning condition of each spinning device depending on the tensile properties measured with the tensile properties measuring section.

With this textile machine, the spinning condition of each spinning device is determined depending on the tensile properties of the yarn measured with the yarn winding device. This configuration makes it possible to identify a spinning device that has caused trouble on a yarn of the yarn supplying bobbin and stop feeding the yarn supplying bobbin from the spinning device to the automatic winder, and adjust the spinning device properly.

### Advantageous Effects of Invention

According to the present invention, tensile properties of a yarn can be measured readily and accurately.

### Brief Description of Drawings

[Fig. 1] Fig. 1 is a configuration diagram of a textile machine of an embodiment of the present invention.
[Fig. 2] Fig. 2 is a perspective view of a yarn supplying bobbin to be conveyed in the textile machine in Fig. 1.
[Fig. 3] Fig. 3 is a front view of a winding unit of the textile machine in Fig. 1.
[Fig. 4] Fig. 4 is a perspective view of a tension sensor of the winding unit in Fig. 3.
[Fig. 5] Fig. 5 is a plan view of the tension sensor taken along line V-V in Fig. 4.
[Fig. 6] Fig. 6 is a front view of the tension sensor in Fig. 4.
[Fig. 7] Fig. 7 is a plan view of the tension sensor in Fig. 4.

### Description of Embodiments

The following describes a preferred embodiment of the present invention in detail with reference to the accompanying drawings. Note that, the same components are denoted by the same reference signs in each drawing, and redundant descriptions are omitted.

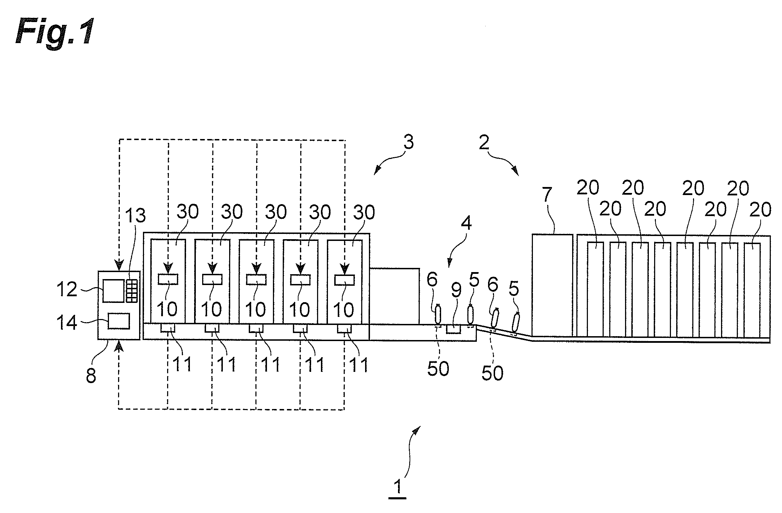

As illustrated in Fig. 1, a textile machine 1 includes a spinning frame 2, an automatic winder 3, and a bobbin feeder 4. The spinning frame 2 includes a plurality of spinning units (spinning devices) 20 each winding a yarn around a spinning tube 5 to form a spinning bobbin 6. The automatic winder 3 includes a plurality of winding units (yarn winding devices) 30 each winding a yarn from the yarn supplying bobbin 6 to form a package. The bobbin feeder 4 feeds such yarn supplying bobbins 6 from the spinning frame 2 to the automatic winder 3. More specifically, the bobbin feeder 4 conveys the yarn supplying bobbin 6 set on a tray 50 from the spinning frame 2 to the automatic winder 3 and conveys the spinning tube 5 set on the tray 50 from the automatic winder 3 to the spinning frame 2.

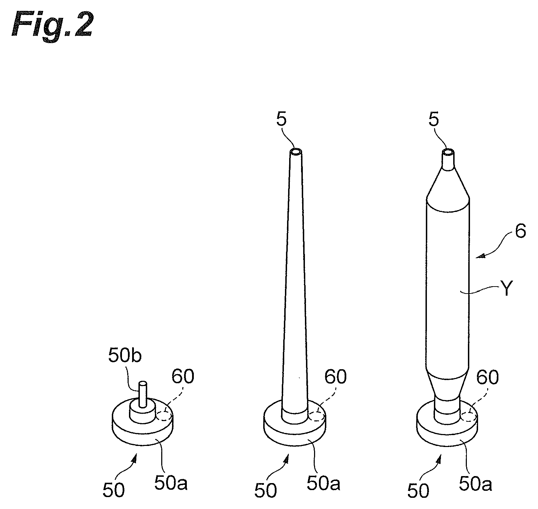

In the textile machine 1, the spinning tube 5 and the yarn supplying bobbin 6 are set on the tray 50 and conveyed in that state. As illustrated in Fig. 2, the tray 50 includes a base 50a formed in a disc and a bobbin plug 50b protruded almost vertically from the base 50a. The spinning tube 5 is formed in a long and narrow tube. The spinning tube 5 is set on the tray 50 when the plug 50b is inserted in the spinning tube 5 from the bottom portion thereof. The yarn supplying bobbin 6 is a bobbin prepared by winding a yarn Y around the spinning tube 5 in the spinning unit 20. Hereinafter, the bobbin around which the yarn Y is wound with the spinning frame 2 is referred to as an actual bobbin.

The textile machine 1 is applied with a textile machine management system using RFID (a radio frequency identification: individual identification using radio waves) technique to manage the condition of the yarn supplying bobbin 6 set on the tray 50. More specifically, the base 50a of each tray 50 includes therein an RF tag (recording unit) 60. In the textile machine 1, information regarding the yarn supplying bobbin 6 is written in the RF tag 60 in each tray 50. With this configuration, the condition of the yarn supplying bobbin 6 is managed.

As illustrated in Fig. 1, the spinning frame 2 is provided with a control device 7 controlling the entire spinning frame 2. The spinning units 20 are arranged side-by-side in a single row. Each of the spinning units 20 twists a roved yarn produced with a rover in a previous stage to perform spinning. In other words, each spinning unit 20 twists a roved yarn stretched and winds the yarn with a predetermined length around the spinning tube 5 to form the yarn supplying bobbin 6 as the actual bobbin.

The spinning frame 2 is configured as what is called a simultaneous doffer, that is, the spinning frame 2 stores a large number of spinning tubes 5 returned from the automatic winder 3 with the bobbin feeder 4 and sets the spinning tubes 5 on the respective spinning units 20 simultaneously at a predetermined timing to wind yarns concurrently. Upon completing the wind of yarns, the spinning frame 2 doffs all the yarn supplying bobbins 6 at the same time, pulls out the stored spinning tubes 5 from such trays 50, and sets the doffed yarn supplying bobbins 6 on the trays 50. Thereafter, the spinning frame 2 sets the spinning tubes 5 that have been pulled out on the respective spinning units 20 again at the same time.

Upon receiving the yarn supplying bobbins 6 from the spinning frame 2, the bobbin feeder 4 writes information regarding the yarn supplying bobbins 6 in such RF tags 60 in the trays 50 on which the yarn supplying bobbins 6 are set. Subsequently, the bobbin feeder 4 assigns the yarn supplying bobbins 6 set on the trays 50 to the respective winding units 30 to feed the bobbins to the automatic winder 3. Writing the information regarding the yarn supplying bobbins 6 is performed by an RF writer (writing unit) 9 arranged on a predetermined position along a conveying path from the spinning frame 2 to the automatic winder 3.

The information regarding the yarn supplying bobbins 6 includes spindle number information used to identify the spinning units 20 that formed yarn supplying bobbins 6 and doffing information used to identify a timing of simultaneous doffing. The yarn supplying bobbins 6 doffed at the same time with the spinning frame 2 are set on the trays 50. Subsequently, the yarn supplying bobbins 6 pass by the RF writer 9 in the same order of the spinning units 20. Thus, the RF writer 9 can identify the spinning units 20 that formed the yarn supplying bobbins 6 by counting the order of the yarn supplying bobbins 6 passing thereby. With this configuration, the RF writer 9 writes the doffing information indicating the time at which simultaneous doffing is performed or the number of times of simultaneous doffing and the spindle number information indicating a spinning unit 20 performed the formation to the RF tag 60 in the tray 50.

The automatic winder 3 is provided with a control device 8 controlling the entire automatic winder 3. The winding units 30 are arranged side-by-side in a single row. Each of the winding units 30 is provided with a control section 10 controlling the winding unit 30 and an RF reader (reading unit) 11 reading information regarding the yarn supplying bobbins 6. The control section 10 receives and transmits various pieces of information regarding winding operation from and to the controller device 8. After the bobbin feeder 4 feeds the winding unit 30 with the yarn supplying bobbins 6, the RF reader 11 reads information regarding the yarn supplying bobbins 6 from the RF tags 60 in the trays 50 on which the yarn supplying bobbins 6 are set and transmits the information to the control device 8. Note that the control device 8 includes a display 12 used to display the condition of each of the winding units 30 or other pieces of information, input keys 13 operated by the operator to, for example, configure winding condition, and a managing section 14 storing and managing various kinds of data.

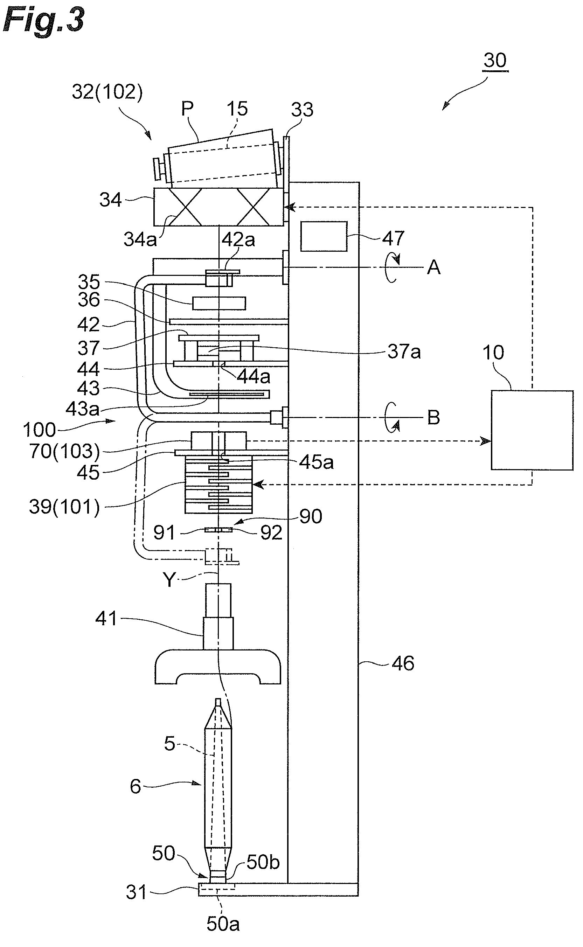

As illustrated in Fig. 3, the winding unit 30 includes a bobbin supporting section 31 supporting the standing yarn supplying bobbin 6 fed by the bobbin feeder 4, and a winding section 32 winding the yarn Y around a winding tube 15 to form a package P, the yarn Y unwound from the yarn supplying bobbin 6 supported with the bobbin supporting section 31. In the present embodiment, hereinafter, a tube used for winding the yarn Y is referred to as a winding tube 15, and the winding tube 15 around which the yarn Y is wound is referred to as a package P. Thus, a package P in which a specific amount of the yarn Y is wound around the winding tube 15 is referred to as a full-bobbin package P.

The winding section 32 includes a winding drum 34 on which drum grooves 34a are formed and a cradle 33 rotatably supporting the winding tube 15. The cradle 33 brings the surface of the yarn Y wound around the winding tube 15 (in other words, the surface of the package P) into contact with the surface of the winding drum 34 with a proper pressure. In the winding section 32, a motor rotationally drives the winding drum 34 to rotate the winding tube 15 so that the yarn Y is wound around the winding tube 15 while the yarn Y is traversed in a predetermined span.

At a position between the bobbin supporting section 31 and the winding section 32, a yarn unwinding assisting device 41, a pre-clearer (yarn defect passing regulator) 90, gate-type tenser (tension applying section) 39, a tension sensor 70, a splicer 37, a cutter 36, and a yarn clearer 35 are provided in this order along the running path of the yarn Y from the lower part (upstream side). Furthermore, a relay pipe 42 and an upper yarn guiding member 43 for splicing are provided thereto. Each of the sections mentioned above are supported by a machine base 46. The machine base 46 is provided with a display 47 displaying the condition of the winding unit 30 or other pieces of information.

The yarn clearer 35 detects a yarn defect, such as a slab, when the yarn Y is being wound. The pre-clearer 90 regulates passage of a yarn defect beforehand, which is larger than a reference value, with a pair of regulating members 91 and 92 arranged in both sides of the running yarn Y with a predetermined gap. The cutter 36 cuts the yarn Y when the yarn clearer 35 detects a yarn defect or the pre-clearer 90 regulates the passage of a yarn defect. The splicer 37 splices yarn ends of a lower yarn from the yarn supplying bobbin 6 and an upper yarn to the package P when the cutter 36 cuts the yarn Y or the yarn Y is broken. The tension sensor 70 measures tension of the yarn Y to be wound with the winding section 32. The gate-type tenser 39 applies predetermined tension to the running yarn Y by holding the yarn Y in zigzags with a pair of gates including a comb-like fixed gate and a comb-like movable gate. The yarn unwinding assisting device 41 uses a tubular member arranged over the yarn supplying bobbin 6 to control a balloon of the yarn Y unwound from the yarn supplying bobbin 6.

The bottom end of the splicer 37 is provided with a guiding plate 44 for a splicer on which a guiding groove 44a for guiding the yarn Y to the splicer 37 is formed. The upper end of the gate-type tenser 39 is provided with a guiding plate 45 for a tenser on which a guiding groove 45a for guiding the yarn Y to the gate-type tenser 39 is formed. The tension sensor 70 is provided between the gate-type tenser 39 and the splicer 37 and mounted on the top surface of the guiding plate 45 for a tenser.

The upper yarn guiding member 43 is configured to be vertically rotatable about a pivot shaft A and has a drawing port 43a at the rotary end thereof. With this configuration, the drawing port 43 a rotates from a vacant room under the splicer 37 to a vacant room over the winding drum 34. The relay pipe 42 is configured to be vertically rotatable about a pivot shaft B and has a drawing port 42a at the rotary end thereof. With this configuration, the drawing port 42a rotates from a vacant room over the splicer 37 to a vacant room under the pre-clearer 90.

When the cutter 36 cuts the yarn Y, the drawing port 43a of the upper yarn guiding member 43 arranged over the winding drum 34 draws the upper yarn, the yarn end for the package P. In this state, the drawing port 43a rotates downward and the yarn end of the upper yarn is passed to the splicer 37. The drawing port 42a of the relay pipe 42 arranged under the pre-clearer 90 draws the lower yarn, the yarn end for the yarn supplying bobbin 6. In this state, the drawing port 42a rotates upward, and the yarn end of the lower yarn is passed to the splicer 37. In this manner, the upper and lower yarns are spliced in the splicer 37.

The splicer 37 is provided with a yarn gathering lever 37a to gather the upper and lower yarns in a site for splicing in order to splice the yarns securely. When splicing is performed, the yarn gathering lever 37a securely introduces the upper and lower yarns to the site for splicing in the splicer 37. The yarn gathering lever 37a also securely introduces a yarn to the tension sensor 70 arranged under the splicer 37.

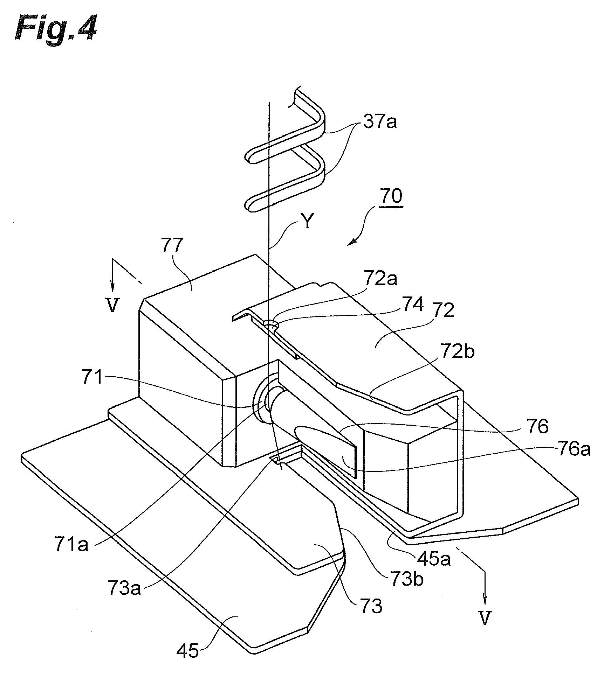

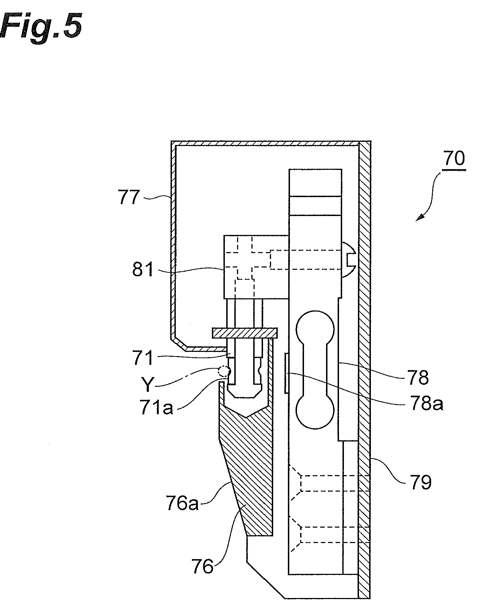

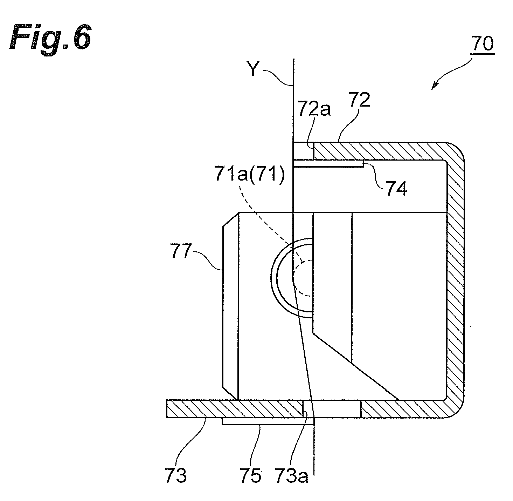

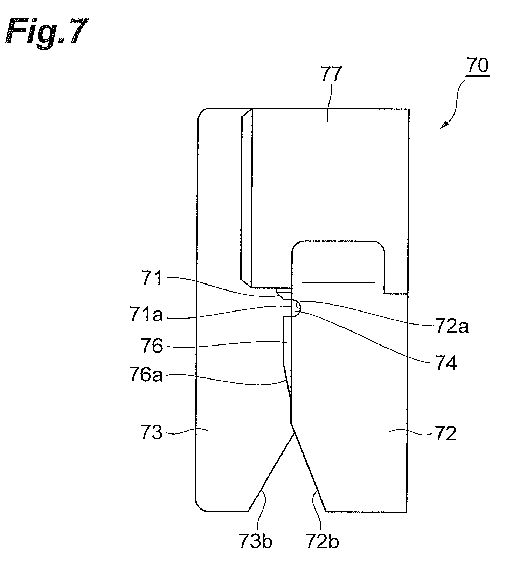

The following describes the tension sensor 70 more specifically. As illustrated in Figs. 4, 5, 6, and 7, the tension sensor 70 includes a rod 71 like a rod. The rod 71 is disposed so that the axial direction thereof is perpendicular to the running path of the yarn Y A side face on the rod 71 is provided with a tension detector 71a detecting the tension of the yarn Y The tension detector 71a has a shape as the periphery of the rod 71 is squeezed. The tension detector 71 a is made from a material such as a ceramic having small contact friction resistance to the yarn Y and less-wearing quality.

An upper guide member 72 supporting the yarn Y over the tension detector 71 a is arranged over the rod 71. The upper guide member 72 is a plate-like member disposed so that the principal surface thereof is perpendicular to the running path of the yarn Y In the part on the upper guide member 72 through which the yarn Y runs, a yarn supporting section 72a is formed as a groove. A yarn guide 74 is attached to the yarn supporting section 72a. The yarn guide 74 is made from a ceramic and brought into contact with the yarn Y from one side. A lower guide member 73 supporting the yarn Y under the tension detector 71 a is arranged under the rod 71. The lower guide member 73 is a plate-like member and is disposed so that the principal surface thereof is perpendicular to the running path of the yarn Y In the part on the lower guide member 73 through which the yarn Y runs, a yarn supporting section 73a is formed as a groove. A yarn guide 75 is attached to the yarn supporting section 73a. The yarn guide 75 is made from a ceramic and brought into contact with the yarn Y from another side.

In this manner, the yarn Y is suspended with the three points of the yarn guide 74 on the upper guide member 72, the yarn guide 75 on the lower guide member 73, and the tension detector 71a on the rod 71. In this case, the yarn Y is bent at a predetermined angle at the tension detector 71 a. Thus, the yarn Y is wound around the tension detector 71a and comes in contact with the tension detector 71a.

A guide section 72b guiding the yarn Y to the tension detector 71a is provided to the upper guide member 72. The guide section 72b has the inclined side inclined one side toward the near side. A guide section 73b guiding the yarn Y to the tension detector 71a is provided to the lower guide member 73. The guide section 73b has the inclined side inclined another side toward the near side. A guide member 76 guiding the yarn Y to the tension detector 71a is provided at the tip end of the rod 71. On the side surface of the tip-end of the guide member 76, a guide surface 76a inclined substantially similar to the guide section 72b of the upper guide member 72 in plan view.

The tension sensor 70 has a body cover 77 in which a metal block (strain body) 78 provided with a resistance element (strain gauge) 78a is arranged. The metal block 78 is shaped in a cuboid and is disposed so that the longitudinal direction of the cuboid is substantially parallel to the axial direction of the rod 71. One end of the metal block 78 is fixed on a supporting section 79 of the tension sensor 70. The other end of the metal block 78 is connected to the base end of the rod 71 through a connecting body 81. The resistance element 78a is formed on the side surface of the metal block 78 integrally by printing or other techniques. With this configuration, the rod 71 is displaced in the direction perpendicular to the axial direction when the yarn Y comes in contact with the tension detector 71 a. This causes strain on the metal block 78 in the longitudinal direction. The resistance element 78a detects the strain and outputs a tension value in the form of a detection signal (voltage signal) associated with the amount of the strain.

As illustrated in Fig. 3, the control section 10 performs feedback-control of the gate-type tenser 39 so as to keep the optimum tension that would cause no yarn breaking or the like, based on the tension value received from the tension sensor 70. The control section 10 may control the winding drum 34 to change the peripheral speed of the package P in addition to the control of the gate-type tenser 39 in order to adjust the tension of the yarn Y to be wound with the winding section 32.

Each winding unit 30 configured as mentioned above can transit to a yarn tensile properties measurement mode in accordance with the operation by the operator using the control device 8 and the control section 10. The yarn tensile properties measurement mode is a mode used in the following cases: for example, when the adjustment of the splicer 37 is finished; when the tensile properties of the spliced part that the splicer 37 spliced is measured regularly in operation of the winding unit 30 to confirm the stability thereof; and when the tensile properties of the yarn itself is measured to confirm the quality of the yarn Y that the spinning unit 20 spun.

In the yarn tensile properties measurement mode, a tensile properties measuring section 100 is organized for each of the winding units 30 as illustrated in Fig. 3. The tensile properties measuring section 100 measures the tensile properties of the yarn Y (tenacity of the yarn Y, yarn tenacity, for example) drawn from the yarn supplying bobbin 6 between the bobbin supporting section 31 and the winding section 32. The tensile properties measuring section 100 includes a holding section 101 holding the yarn Y drawn from the yarn supplying bobbin 6, a stretching section 102 stretching the yarn Y held with the holding section 101, and a tensile properties detecting section 103 detecting the tensile properties of the yarn Y between the holding section 101 and the stretching section 102. In the winding unit 30, the gate-type tenser 39 functions as the holding section 101, the winding section 32 functions as the stretching section 102, and the tension sensor 70 functions as the tensile properties detecting section 103.

When the gate-type tenser 39 serves as the holding section 101, the yarn Y is securely held with the gate-type tenser 39 to prevent the yarn Y from deviating. This operation differs from that in winding of the yarn Y More specifically, compared with a state in which the gate-type tenser 39 holds the yarn Y in zigzags, the movable gate further moves toward the fixed gate. In this way, the yarn Y can be clamped between the movable gate and the fixed gate. When the winding section 32 serves as the stretching section 102, in order to ensure friction force higher than a predetermined value, the surface of the package P is brought into contact with the surface of the winding drum 34 with higher pressure than that of the winding of the yarn Y. In addition to that, the winding drum 34 is rotated by higher torque than that of winding of the yarn Y and the rotation speed of the winding drum 34 at that time is lower than that of the winding of the yarn Y When the tension sensor 70 functions as the tensile properties detecting section 103, the amount of strain on the yarn Y at a predetermined timing, such as yarn breaking, is converted to a load value to detect the tensile properties of the yarn Y in grams. Note that the pre-clearer 90 may function as the holding section 101. In this case, the pair of regulating members 91 and 92 of the pre-clearer 90 is configured to be movable so as to securely clamp the yarn Y with the pair of regulating members 91 and 92.

As described above, in the winding unit 30, the tensile properties of the yarn Y drawn from the yarn supplying bobbin 6 is measured with the tensile properties measuring section 100 between the bobbin supporting section 31 and the winding section 32. In this manner, the tensile properties of the yarn Y can be measured in the winding unit 30. Thus, the conventional complicated operation in which sampling of a yarn is performed two or more times on each winding unit or the tensile properties of the yarn is measured with a detector separately prepared is unnecessary. With the winding unit 30, the tensile properties of the yarn Y therefore can be measured readily and accurately. In the automatic winder 3 including a plurality of winding units 30 and the textile machine 1 including the automatic winder 3, each of the winding units 30 includes the tensile properties measuring unit 100. Thus, similar to the above, the tensile properties of the yarn Y can be measured readily and accurately.

In the tensile properties measuring section 100, the yarn Y drawn from the yarn supplying bobbin 6 is held with the holding section 101 and also stretched with the stretching section 102, and the tensile properties of the yarn Y is detected with the tensile properties detecting section 103 between the holding section 101 and the stretching section 102. In this manner, when detecting the tensile properties of the yarn Y, the yarn Y drawn from the yarn supplying bobbin 6 is held with the holding section 101. Thus, the tensile properties measured part can be prevented from being wound into the package P (in other words, it is possible to prevent the formation of the package P containing the tensile properties measured part).

In the winding unit 30, the gate-type tenser 39 (optionally, the pre-clearer 90) functions as the holding section 101 of the tensile properties measuring section 100, the winding section 32 functions as the stretching section 102 of the tensile properties measuring section 100, and the tension sensor 70 functions as the tensile properties detecting section 103 of the tensile properties measuring section 100. As described above, the structure used in the winding of the yarn Y is also used in the yarn tensile properties measurement mode. Thus, the number of components of the winding unit 30 can be reduced to simplify the structure of the winding unit 30.

The tensile properties measuring section 100 is configured to measure the breaking intensity (breaking tension) of the yarn Y. In this case, the tensile properties measurement breaks the yarn Y Thus, the tensile properties measured part can be securely prevented from being wound into the package P (in other words, it is possible to prevent the formation of the package P containing the tensile properties measured part). Note that the tensile properties measuring section 100 is not limited to an instrument that measures the breaking intensity of the yarn Y. The tensile properties measuring section 100, for example, may set a reference value within a range of measuring tension for the tension sensor 70 and determine whether the tensile properties of the yarn Y is higher than the reference value.

The tensile properties measuring section 100 is configured to measure the tensile properties of the yarn Y in the state with a spliced part spliced with the splicer 37. In this case, the tensile properties of the yarn Y with a spliced part can be managed numerically and thus, the quality control of the spliced part can be simplified. Furthermore, the splicer 37 may be adjusted on the basis of the tensile properties of the yarn Y with a spliced part so as to splice yarns with proper tensile properties.

In addition to that, the tensile properties measuring section 100 is configured to measure the tensile properties of the yarn Y in the state without a spliced part spliced with the splicer 37. In this case, it is possible to guess which, the tensile properties of the spliced part or the tensile properties of the yarn Y itself, causes the tensile properties of the yarn Y with the spliced part.

The display 47 is configured to display the tensile properties measured with the tensile properties measuring section 100. In this case, the operator can quickly confirm the tensile properties of the yarn Y Furthermore, after the operator adjusts the winding unit 30 or performs other tasks, the operator can understand whether the setting-up through the adjustment or the like is proper. Note that the tensile properties measured with the tensile properties measuring section 100 may be displayed on the display 12 of the control device 8.

The management section 14 of the control device 8 in the automatic winder 3 is configured to store and manage the tensile properties measured with the tensile properties measuring section 100. In this case, quality control on the packages P, for example, formed with the winding unit 30 can be practiced advantageously.

The control device 8 in the automatic winder 3 is configured to identify spinning condition in each spinning unit 20 on the basis of the tensile properties measured with the tensile properties measuring section 100 in each of the winding units 30. Note that the control device 8 receives information from the control section 10 in each of the winding units 30, the information indicating the tensile properties (of the yarn Y from the yarn supplying bobbin 6) measured with the tensile properties measuring section 100 in each of the winding units 30. In addition to that, the control device 8 receives information regarding the yarn supplying bobbin 6 (including spindle number information and doffing information) from the RF reader 11 in each of the winding units 30. Thus, the control device 8 can associate the tensile properties of the yarn Y from the yarn supplying bobbin 6 with the spinning unit 20 that spun the yarn Y from the yarn supplying bobbin 6. This configuration makes it possible to identify a spinning device 20 that has caused trouble on the yarn Y from the yarn supplying bobbin 6 and stop feeding the yarn supplying bobbin 6 from the spinning device 20 to the automatic winder 3, and adjust the spinning unit 20 properly.

The present invention has been described above in detail based on an embodiment thereof. However, the present invention is not limited to the embodiment described above. For example, the winding section 32 is not limited to an instrument that performs traversing of the yarn Y with the winding drum 34. The winding section 32 may traverse the yarn Y with a traverse arm, for example. In this case, when a motor rotationally drives the winding tube 15, in the yarn tensile properties measurement mode, a pressure used to press the surface of the package P to the surface of the winding drum 34 is not necessary to be higher than that in winding of the yarn Y The tension applying section is not limited to the gate-type tenser 39. A disk-type tenser may also be used as the tension applying section. As another embodiment in which the winding section 32 functions as the stretching section 102, it is possible that the cradle 33 supporting the package P be applied with a brake to hold the package P not to rotate, and the yarn Y having held be stretched by displacing the cradle 33 upward (lifting up).

### Industrial Applicability

According to the present invention, the tensile properties of the yarn can be measured readily and accurately.

### Reference Signs List

1... textile machine, 2... spinning frame, 3... automatic winder, 4... bobbin feeder, 6... yarn supplying bobbin, 8... control device, 10... control section, 14... management section, 20... spinning unit (spinning device), 30... winding unit (yarn winding device), 31... bobbin supporting section, 32... winding section, 37... splicer, 39... gate-type tenser (tension applying section), 47... display, 70... tension sensor, 90... pre-clearer (yarn defect passing regulator), 91, 92... regulating member, 100... tensile properties measuring section, 101... holding section, 102... stretching section, 103... tensile properties detecting section, Y.. yarn.

## Claims

1. A yarn winding device (30) winding a yarn (Y) from a yarn supplying bobbin (6) to form a package (P), the yarn winding device (30) comprising:
a bobbin supporting section (31) adapted to support the yarn supplying bobbin (6);
a winding section (32) adapted to wind the yarn (Y) from the yarn supplying bobbin (6) supported with the bobbin supporting section (31) and adapted to form the package (P); and
a tensile properties measuring section (100) adapted to measure tensile properties of the yarn (Y) drawn from the yarn supplying bobbin (6), between the bobbin supporting section (31) and the winding section (32);
wherein the tensile properties measuring section (100) is adapted to measure a breaking intensity of the yarn (Y); and
wherein the tensile properties measuring section (100) includes:
a holding section (101) adapted to hold the yarn (Y) drawn from the yarn supplying bobbin (6); and
a stretching section (102) adapted to stretch the yarn (Y) held with the holding section (101);
**characterized in that**
the tensile properties measuring section (100) includes a tensile properties detecting section (103) adapted to detect the tensile properties of the yarn (Y) between the holding section (101) and the stretching section (102);
the yarn winding device (30) further comprises a tension sensor (70) adapted to measure a tension of the running yarn (Y) being wound with the winding section (32); and
the tension sensor (70) is adapted to function as the tensile properties detecting section (103).

2. The yarn winding device (30) according to claim 1, wherein the winding section (32) is adapted to function as the stretching section (102).

3. The yarn winding device (30) according to claim 1 or 2, further comprising a tension applying section (39) adapted to apply tension to the yarn (Y) running, between the bobbin supporting section (31) and the winding section (32); and
wherein the tension applying section (39) is adapted to function as the holding section (101).

4. The yarn winding device (30) according to claim 1 or 2, further comprising a yarn defect passing regulator (90) adapted to regulate passage of a yarn defect that is larger than a reference value, with a pair of regulating members (91, 92) arranged on both sides of the yarn (Y) running, with a predetermined gap, between the bobbin supporting section (31) and the winding section (32); and
wherein the yarn defect passing regulator (90) is adapted to function as the holding section (101) by using the regulating members (91, 92) to hold the yarn (Y).

5. An automatic winder (3) comprising a plurality of yarn winding devices (30) according to any one of claims 1 to 4.

6. The automatic winder (3) according to claim 5, wherein each of the yarn winding devices (30) further includes a splicer (37) adapted to splice the yarn (Y) that has been cut, between the bobbin supporting section (31) and the winding section (32); and
wherein tensile properties measuring section (100) is adapted to measure the tensile properties of the yarn (Y) in the state with a spliced part spliced with the splicer (37).

7. The automatic winder (3) according to claim 5 or 6, wherein each of the yarn winding devices (30) further includes a splicer (37) adapted to splice the yarn (Y) that has been cut, between the bobbin supporting section (31) and the winding section (32); and
wherein the tensile properties measuring section (100) adapted to measure the tensile properties of the yarn (Y) in the state with a spliced part spliced with the splicer (37) and measures the tensile properties of the yarn (Y) in the state without the spliced part.

8. The automatic winder (3) according to any one of claims 5 to 7, further comprising a display (47) adapted to display the tensile properties measured with the tensile properties measuring section (100).

9. The automatic winder (3) according to any one of claims 5 to 9 further comprising a control device (8) adapted to control the yarn winding devices (30); and
wherein the control device (8) includes a managing unit adapted to store and manage the tensile properties measured with the tensile properties measuring section (100).

10. A textile machine comprising:
a spinning frame (2) including a plurality of spinning devices (20) each forming a yarn supplying bobbin (6);
an automatic winder (3) including a plurality of yarn winding devices (30) according to any one of claims 1 to 4, each adapted to wind a yarn (Y) from the yarn supplying bobbin (6) to form a package (P);
a bobbin feeder (4) adapted to feed the yarn supplying bobbin (6) from the spinning frame (2) to the automatic winder (3); and
a control device (8) adapted to control at least the automatic winder (3);
wherein the control device (8) is adapted to identify a spinning
condition of each spinning device (20) depending on the tensile properties measured with the tensile properties measuring section (100).

## Patentansprüche

1. Eine Garnwickelvorrichtung (30), die ein Garn (Y) von einer Garnzufuhrspule (6) wickelt, um einen Wickelkörper (P) zu bilden, wobei die Garnwickelvorrichtung (30) folgende Merkmale aufweist:
einen Spulenträgerabschnitt (31), der angepasst ist, die Garnzufuhrspule (6) zu tragen;
einen Wickelabschnitt (32), der angepasst ist, das Garn (Y) von der Garnzufuhrspule (6), die durch den Spulenträgerabschnitt (31) getragen wird, zu wickeln und angepasst ist, den Wickelkörper (P) zu bilden,; und
einen Zugeigenschaften-Messabschnitt (100), der angepasst ist, Zugeigenschaften des Garns (Y), das von der Garnzufuhrspule (6) gezogen wird, zwischen dem Spulenträgerabschnitt (31) und dem Wickelabschnitt (32) zu messen;
wobei der Zugeigenschaften-Messabschnitt (100) angepasst ist, eine Bruchstärke des Garns (Y) zu messen; und
wobei der Zugeigenschaften-Messabschnitt (100) folgende Merkmale aufweist:
einen Halteabschnitt (101), der angepasst ist, das Garn (Y), das von der Garnzufuhrspule (6) gezogen wird, zu halten; und
einen Dehnabschnitt (102), der angepasst ist, das Garn (Y), das durch den Halteabschnitt (101) gehalten wird, zu dehnen;
**dadurch gekennzeichnet, dass**
der Zugeigenschaften-Messabschnitt (100) einen Zugeigenschaften-Erfassungsabschnitt (103) umfasst, der angepasst ist, die Zugeigenschaften des Garns (Y) zwischen dem Halteabschnitt (101) und dem Dehnabschnitt (102) zu erfassen;
die Garnwickelvorrichtung (30) ferner einen Zugspannungssensor (70) aufweist, der angepasst ist, eine Zugspannung des laufenden Garns (Y), das durch den Wickelabschnitt (32) gewickelt wird, zu messen; und
der Zugspannungssensor (70) angepasst ist, als der Zugeigenschaften-Erfassungsabschnitt (103) zu wirken.

2. Die Garnwickelvorrichtung (30) gemäß Anspruch 1, bei der der Wickelabschnitt (32) angepasst ist, als der Dehnabschnitt (102) zu wirken.

3. Die Garnwickelvorrichtung (30) gemäß Anspruch 1 oder 2, die ferner einen Zugspannung-Ausübungsabschnitt (39) aufweist, der angepasst ist, eine Zugspannung auf das Garn (Y) auszuüben, das zwischen dem Spulenträgerabschnitt (31) und dem Wickelabschnitt (32) verläuft; und
wobei der Zugspannung-Ausübungsabschnitt (39) angepasst ist, als der Halteabschnitt (101) zu wirken.

4. Die Garnwickelvorrichtung (30) gemäß Anspruch 1 oder 2, die ferner einen Garndefekt-Durchgangsregler (90) aufweist, der angepasst ist, einen Durchgang eines Garndefekts, der größer als ein Referenzwert ist, durch ein Paar von Regelbaugliedern (91, 92) zu regeln, die an beiden Seiten des Garns (Y) angeordnet sind, das mit einem vorbestimmten Abstand zwischen dem Spulenträgerabschnitt (31) und dem Wickelabschnitt (32) verläuft; und
wobei der Garndefekt-Durchgangsregler (90) angepasst ist, als der Halteabschnitt (101) zu wirken, indem die Regelbauglieder (91, 92) zum Halten des Garns (Y) verwendet werden.

5. Eine automatische Wickelvorrichtung (3), die eine Mehrzahl von Garnwickelvorrichtungen (30) gemäß einem der Ansprüche 1 bis 4 aufweist.

6. Die automatische Wickelvorrichtung (3) gemäß Anspruch 5, bei der jede der Garnwickelvorrichtungen (30) ferner eine Spleißvorrichtung (37) umfasst, die angepasst ist, das Garn (Y), das abgeschnitten worden ist, zwischen dem Spulenträgerabschnitt (31) und dem Wickelabschnitt (32) zu spleißen; und
wobei der Zugeigenschaften-Messabschnitt (100) angepasst ist, die Zugeigenschaften des Garns (Y) in dem Zustand mit einem gespleißten Teil, das durch die Spleißvorrichtung (37) gespleißt ist, zu messen.

7. Die automatische Wickelvorrichtung (3) gemäß Anspruch 5 oder 6, bei der jede der Garnwickelvorrichtungen (30) ferner eine Spleißvorrichtung (37) umfasst, die angepasst ist, das Garn (Y), das abgeschnitten worden ist, zwischen dem Spulenträgerabschnitt (31) und dem Wickelabschnitt (32) zu spleißen; und
wobei der Zugeigenschaften-Messabschnitt (100) angepasst ist, die Zugeigenschaften des Garns (Y) in dem Zustand mit einem gespleißten Teil, das durch die Spleißvorrichtung (37) gespleißt ist, zu messen, und die Zugeigenschaften des Garns (Y) in dem Zustand ohne das gespleißte Teil misst.

8. Die automatische Wickelvorrichtung (3) gemäß einem der Ansprüche 5 bis 7, die ferner eine Anzeige (47) aufweist, die angepasst ist, die Zugeigenschaften anzuzeigen, die durch den Zugeigenschaften-Messabschnitt (100) gemessen werden.

9. Die automatische Wickelvorrichtung (3) gemäß einem der Ansprüche 5 bis 9, die ferner eine Steuervorrichtung (8) aufweist, die angepasst ist, die Garnwickelvorrichtungen (30) zu steuern; und
wobei die Steuervorrichtung (8) eine Verwaltungseinheit umfasst, die angepasst ist, die Zugeigenschaften, die durch den Zugeigenschaften-Messabschnitt (100) gemessen werden, zu speichern und zu verwalten.

10. Eine Textilmaschine, die folgende Merkmale aufweist:
eine Spinnmaschine (2), die eine Mehrzahl von Spinnvorrichtungen (20) aufweist, die jeweils eine Garnzufuhrspule (6) bilden;
eine automatische Wickelvorrichtung (3), die eine Mehrzahl von Garnwickelvorrichtungen (30) gemäß einem der Ansprüche 1 bis 4 umfasst, die jeweils angepasst sind, ein Garn (Y) von der Garnzufuhrspule (6) zu wickeln, um einen Wickelkörper (P) zu bilden;
eine Spulenzuführungsvorrichtung (4), die angepasst ist, die Garnzufuhrspule (6) von der Spinnmaschine (2) zu der automatischen Wickelvorrichtung (3) zuzuführen; und
eine Steuervorrichtung (8), die angepasst ist, zumindest die automatische Wickelvorrichtung (3) zu steuern;
wobei die Steuervorrichtung (8) angepasst ist, von jeder Spinnvorrichtung (20) abhängig von den Zugeigenschaften, die durch den Zugeigenschaften-Messabschnitt (100) gemessen werden, eine Spinnbedingung zu identifizieren.

## Revendications

1. Dispositif de bobinage de fil (30) enroulant un fil (Y) d'une bobine d'alimentation de fil (6) pour former un paquet (P), le dispositif de bobinage de fil (30) comprenant:
un segment de support de bobine (31) adapté pour supporter la bobine d'alimentation de fil (6);
un segment de bobinage (32) adapté pour enrouler le fil (Y) de la bobine d'alimentation de fil (6) supportée par le segment de support de bobine (31) et adaptée pour former le paquet (P); et
un segment de mesure de propriétés de traction (100) adapté pour mesurer les propriétés de traction du fil (Y) tiré de la bobine d'alimentation de fil (6), entre le segment de support de bobine (31) et le segment de bobinage (32);
dans lequel le segment de mesure de propriétés de traction (100) est adapté pour mesurer une intensité de rupture du fil (Y); et
dans lequel le segment de mesure de propriétés de traction (100) comporte:
un segment de maintien (101) adapté pour maintenir le fil (Y) tiré de la bobine d'alimentation de fil (6); et
un segment d'étirage (102) adapté pour étirer le fil (Y) maintenu par le segment de maintien (101);
**caractérisé par le fait que**
le segment de mesure de propriétés de traction (100) comporte un segment de détection de propriétés de traction (103) adapté pour détecter les propriétés de traction du fil (Y) entre le segment de maintien (101) et le segment d'étirage (102);
le dispositif de bobinage de fil (30) comprend par ailleurs un capteur de tension (70) adapté pour mesurer une tension du fil en déplacement (Y) enroulé par le segment de bobinage (32); et
le capteur de tension (70) est adapté pour fonctionner comme segment de détection de propriétés de traction (103).

2. Dispositif de bobinage de fil (30) selon la revendication 1, dans lequel le segment de bobinage (32) est adapté pour fonctionner comme segment d'étirage (102).

3. Dispositif de bobinage de fil (30) selon la revendication 1 ou 2, comprenant par ailleurs un segment d'application de tension (39) adapté pour appliquer une tension sur le fil (Y) en déplacement, entre le segment de support de bobine (31) et le segment de bobinage (32); et
dans lequel le segment d'application de tension (39) est adapté pour fonctionner comme segment de maintien (101).

4. Dispositif de bobinage de fil (30) selon la revendication 1 ou 2, comprenant par ailleurs un régulateur de passage de défaut de fil (90) adapté pour réguler le passage d'un défaut de fil qui est supérieur à une valeur de référence, avec une paire d'éléments de régulation (91, 92) disposés des deux côtés du fil (Y) en déplacement, à un intervalle prédéterminé, entre le segment de support de bobine (31) et le segment de bobinage (32); et
dans lequel le régulateur de passage de défaut de fil (90) est adapté pour fonctionner comme segment de maintien (101) à l'aide des éléments de régulation (91, 92) pour maintenir le fil (Y).

5. Bobinoir de fil automatique (3) comprenant une pluralité de dispositifs de bobinage de fil (30) selon l'une quelconque des revendications 1 à 4.

6. Bobinoir de fil automatique (3) selon la revendication 5, dans lequel chacun des dispositifs de bobinage de fil (30) comporte par ailleurs un dispositif d'épissage (37) adapté pour épisser le fil (Y) qui a été coupé, entre le segment de support de bobine (31) et le segment de bobinage (32); et
dans lequel le segment de mesure de propriétés de traction (100) est adapté pour mesurer les propriétés de traction du fil (Y) à l'état avec une partie épissée par le dispositif d'épissage (37).

7. Bobinoir de fil automatique (3) selon la revendication 5 ou 6, dans lequel chacun des dispositifs de bobinage de fil (30) comporte par ailleurs un dispositif d'épissage (37) adapté pour épisser le fil (Y) qui a été coupé, entre le segment de support de bobine (31) et le segment de bobinage (32); et
dans lequel le segment de mesure de propriétés de traction (100) est adapté pour mesurer les propriétés de traction du fil (Y) à l'état avec une partie épissée par le dispositif d'épissage (37) et mesure les propriétés de traction du fil (Y) à l'état sans la partie épissée.

8. Bobinoir de fil automatique (3) selon l'une quelconque des revendications 5 à 7, comprenant par ailleurs un dispositif d'affichage (47) adapté pour afficher les propriétés de traction mesurées par le segment de mesure de propriétés de traction (100).

9. Bobinoir de fil automatique (3) selon l'une quelconque des revendications 5 à 9, comprenant par ailleurs un dispositif de commande (8) adapté pour commander les dispositifs de bobinage de fil (30); et
dans lequel le dispositif de commande (8) comporte une unité de gestion adaptée pour mémoriser et gérer les propriétés de traction mesurées par le segment de mesure de propriétés de traction (100).

10. Machine textile comprenant:
un métier à filer (2) comportant une pluralité de dispositifs de filage (20) formant, chacun, une bobine d'alimentation de fil (6);
un bobinoir de fil automatique (3) comportant une pluralité de dispositifs de bobinage de fil (30) selon l'une quelconque des revendications 1 à 4, adaptés, chacun, pour enrouler un fil (Y) de la bobine d'alimentation de fil (6) pour former un paquet (P);
un dispositif d'alimentation de bobine (4) adapté pour alimenter la bobine d'alimentation de fil (6) du métier à filer (2) vers le bobinoir de fil automatique (3); et
un dispositif de commande (8) adapté pour commander au moins le bobinoir de fil automatique (3);
dans lequel le dispositif de commande (8) est adapté pour identifier une condition de filage de chaque dispositif de filage (20) en fonction des propriétés de traction mesurées par le segment de mesure de propriétés de traction (100).
